# EUROPEAN PATENT APPLICATION

(11) **EP 4 682 257 A1**
(43) Date of publication of application: **21.01.2026**
(21) Application number: 24774851.0
(22) Date of filing: 15.03.2024
(51) Int. Cl.: C12N 15/12, C12N 1/15, C12N 1/19, C12N 1/21, C12N 5/10, C12N 15/63, C12Q 1/02

(54) **POLYNUCLEOTIDE**

(30) Priority: 17.03.2023 JP 2023043262
(71) Applicant: Sumitomo Chemical Company, Limited, Tokyo 103-6020 (JP)
(72) Inventor: SASAOKA, Norio, Osaka-shi, Osaka 554-8558 (JP); TAKAHASHI, Yasuhiko, Osaka-shi, Osaka 554-8558 (JP); KOBAYASHI, Kentaro, Osaka-shi, Osaka 554-8558 (JP)
(74) Representative: Vossius & Partner Patentanwälte Rechtsanwälte mbB
(86) International application number: PCT/JP2024/010192
(87) International publication number: WO 2024/195715

(57) **Abstract**

A polynucleotide is provided that enables chemical response activity with enhanced stability regardless of the type of sensor protein while containing coding sequences for multiple proteins, including a sensor protein. The polynucleotide contains an expression cassette 1, which contains only a coding sequence of a sensor protein as a protein-coding sequence, and an expression cassette 2, which contains at least two coding sequences for proteins.

## Description

### Technical Field

The present invention relates to polynucleotides and the like.

### Background Art

Groups of odorous substances associated with specific human diseases and mental states have been identified. Owing to their high value as diagnostic markers, there has been growing interest in developing various odor sensors that target these substances. Biological odorant receptors exhibit excellent characteristics, including diversity, sensitivity, and selectivity, which are not found in conventional odor sensor elements such as semiconductors; thus, these odorant receptors offer a promising foundation for the development of novel odor sensors using them as sensor elements.

PTL 1 discloses the use of cells expressing modified odorant receptors or lipid bilayer membranes incorporating modified odorant receptors as odor sensors.

### Citation List

### Patent Literature

PTL 1: WO2022/024902A

### Summary of Invention

### Technical Problem

Odor sensors that rely on the process of artificially preparing lipid bilayer membranes equipped with sensor proteins (e.g., odorant receptors) are often inefficient to produce, and there has been demand for further improvement in the production efficiency of odor sensors. The inventors then focused on using cells that express sensor proteins.

Producing sensor cells that stably express a sensor protein typically requires incorporating not only the sequence of the sensor protein, but also other sequences coding proteins such as those necessary for detecting chemical substances by the sensor protein (e.g., chromogenic or luminescent proteins, and olfactory receptor co-receptors) and drug-resistant genes necessary for cellular drug screening into the genomic DNA of the cells. In PTL 1, the coding sequences of these proteins are each loaded onto individual vectors and introduced into cells.

In the course of their research, the present inventors focused on the following two points. From the perspective of increased efficiency in incorporating multiple protein-coding sequences into the genomic DNA in a given cell, it is desirable for the multiple protein-coding sequences to be carried on a single vector. Furthermore, from the perspective of reducing vector size by limiting the number of promoters and terminators, thereby enhancing the efficiency of vector delivery into cells, it is desirable to incorporate multiple protein-coding sequences into a single expression cassette.

However, during their continued research, the inventors discovered that incorporating a sensor protein-coding sequence and coding sequences of other proteins into a single expression cassette results in the position of the sensor protein-coding sequence affecting chemical response activity; that this impact varies depending on the type of sensor protein; and that some types of sensor proteins, depending on their position, may not exhibit chemical response activity.

From the perspective of sensor use in disease diagnosis and other similar purposes, it is desirable to produce and use multiple types of cells that express mutually different sensor proteins. To achieve this goal, it is important to enable chemical response activity with enhanced stability regardless of the type of sensor protein.

An object of the present disclosure is to provide a polynucleotide that enables chemical response activity with enhanced stability regardless of the type of sensor protein, while including coding sequences for multiple proteins, including a sensor protein.

### Solution to Problem

In light of the object above, the present inventors diligently conducted research and discovered that the object can be achieved by a polynucleotide containing an expression cassette 1, which contains only a coding sequence of a sensor protein as a protein-coding sequence, and an expression cassette 2, which contains at least two protein-coding sequences. The present inventors conducted further research based on this finding and completed the invention of the present disclosure. Specifically, the present disclosure encompasses the following embodiments.

### Item 1.

A polynucleotide comprising
an expression cassette 1 containing only a coding sequence for a sensor protein as a protein-coding sequence, and
an expression cassette 2 containing at least two coding sequences for proteins.

### Item 2.

The polynucleotide according to Item 1, wherein the sensor protein is an odorant receptor protein.

### Item 3.

The polynucleotide according to Item 1 or 2, wherein the sensor protein is an insect odorant receptor protein.

### Item 4.

The polynucleotide according to any one of Items 1 to 3, wherein the expression cassette 2 contains at least two coding sequences selected from the group consisting of a coding sequence for a chromogenic or luminescent protein, a coding sequence for a drug-resistant gene, and a coding sequence for an olfactory receptor co-receptor.

### Item 5.

The polynucleotide according to any one of Items 1 to 4, wherein the expression cassette 2 contains a coding sequence for a chromogenic or luminescent protein, a coding sequence for a drug-resistant gene, and a coding sequence for an olfactory receptor co-receptor.

### Item 6.

The polynucleotide according to any one of Items 1 to 5, wherein the expression cassette 2 has a self-cleaving peptide-coding sequence inserted between the coding sequences.

### Item 7.

The polynucleotide according to any one of Items 1 to 6, which is a vector.

### Item 8.

A cell comprising the polynucleotide of any one of Items 1 to 7.

### Item 9.

A cell chip comprising one or more compartments each containing the cell of Item 8.

### Item 10.

The cell chip according to Item 9, wherein the cell includes two or more types of cells containing mutually different types of the sensor protein.

### Item 11.

The cell chip according to Item 9 or 10, for use in the detection of a chemical substance.

### Advantageous Effects of Invention

The present disclosure provides a polynucleotide that enables chemical response activity with enhanced stability regardless of the type of sensor protein, while including coding sequences for multiple proteins, including a sensor protein, and also provides a cell containing the polynucleotide, a cell chip containing the cell, and so on.

### Brief Description of Drawings

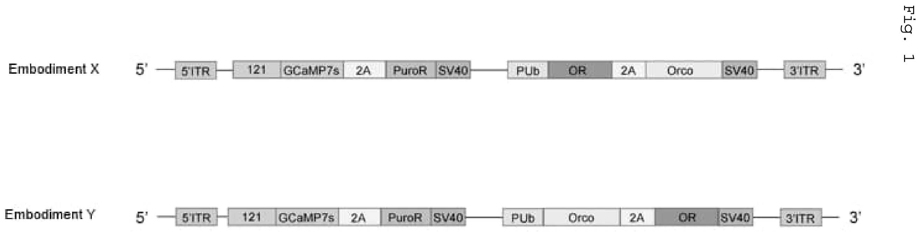

Fig. 1 shows the structure of the vector in embodiment X and the vector in embodiment Y prepared in Test Example 1.

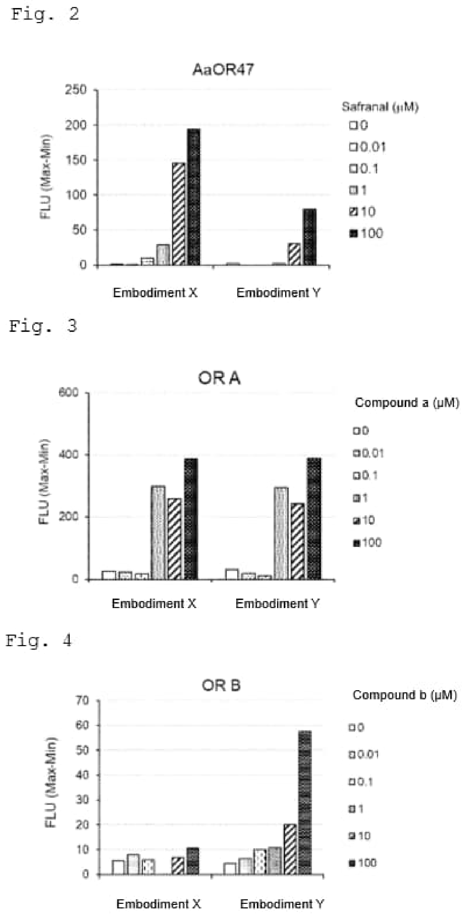

Fig. 2 shows the chemical response activity measured in Test Example 1. The vertical axis indicates the difference in fluorescence intensity before and after the addition of a chemical substance. The horizontal axis indicates the embodiment of the vector used. The legend shows the concentration of the chemical substance. The odorant receptor expressed by the cells is shown at the top of the graph.
Fig. 3 shows the chemical response activity measured in Test Example 1. The vertical axis indicates the difference in fluorescence intensity before and after the addition of a chemical substance. The horizontal axis indicates the embodiment of the vector used. The legend shows the concentration of the chemical substance. The odorant receptor expressed by the cells is shown at the top of the graph.
Fig. 4 shows the chemical response activity measured in Test Example 1. The vertical axis indicates the difference in fluorescence intensity before and after the addition of a chemical substance. The horizontal axis indicates the embodiment of the vector used. The legend shows the concentration of the chemical substance. The odorant receptor expressed by the cells is shown at the top of the graph.

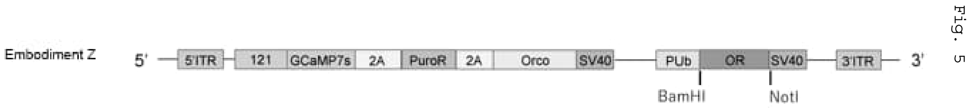

Fig. 5 shows the structure of the vector in embodiment z prepared in Test Example 2.

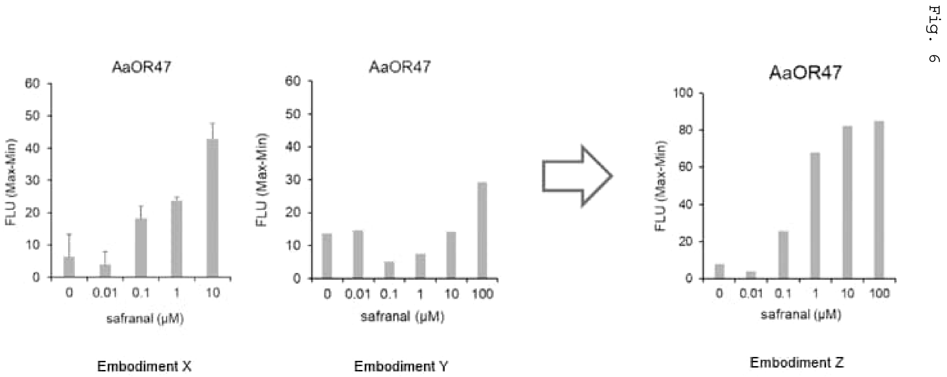

Fig. 6 shows the chemical response activity measured in Test Example 2. The vertical axis indicates the difference in fluorescence intensity before and after the addition of a chemical substance. The horizontal axis indicates the concentration of the chemical substance. The odorant receptor expressed by the cells is shown at the top of the graph. The embodiment of the vector used is shown at the bottom of each graph.

### Description of Embodiments

In the present specification, the terms "comprising," "containing," and "including" include the concepts of comprising, containing, consisting essentially of, and consisting of.

In an embodiment, the present disclosure relates to a polynucleotide comprising an expression cassette 1 containing only a coding sequence for a sensor protein as a protein-coding sequence, and an expression cassette 2 containing at least two coding sequences for proteins ("the polynucleotide of the present disclosure" in the present specification). The following provides an explanation of this polynucleotide.

The sensor protein, the coding sequence of which is included in the expression cassette 1, may be selected from proteins capable of detecting the presence of a chemical substance, such as a receptor protein that uses a chemical substance as a ligand. The sensor protein is particularly preferably an odorant receptor protein.

The odorant receptor protein is a membrane protein with a seven-transmembrane structure, and functions as an odor sensor in organisms. The odorant receptor protein is composed of the following components that are sequentially linked from the amino terminus ("N-terminus" below) to the carboxyl terminus ("C-terminus" below): N-terminal region (NT), first transmembrane domain (TM1), first extracellular loop (EC1), second transmembrane domain (TM2), first intracellular loop (IC1), third transmembrane domain (TM3), second extracellular loop (EC2), fourth transmembrane domain (TM4), second intracellular loop (IC2), fifth transmembrane domain (TM5), third extracellular loop (EC3), sixth transmembrane domain (TM6), third intracellular loop (IC3), seventh transmembrane domain (TM7), and C-terminal region (CT). In the present disclosure, each region is determined by structural prediction using TMpred (K. Hofmann, W. Stoffel, TMbase - a database of membrane spanning proteins segments, Biol. Chem. Hoppe-Seyler, 374 (1993), p. 166, https://embnet.vital-it.ch/software/TMPRED_form.html) (with default conditions).

From the perspective of suitability for chemical detection, the odorant receptor protein is particularly preferably an insect odorant receptor protein. The insect of origin for the insect odorant receptor protein is preferably an insect in the order *Diptera,* such as the family *Culicidae* and the family *Drosophilidae;* an insect in the order *Lepidoptera,* such as the family *Bombycidae;* an insect in the order *Hymenoptera,* such as the family *Apidae;* an insect in the order *Orthoptera,* such as the family *Acrididae;* and an insect in the order *Hemiptera,* such as the family *Cimicidae,* and more preferably an insect in the order *Diptera,* such as the family *Culicidae* and the family *Drosophilidae;* an insect in the order *Orthoptera,* such as the family *Acrididae;* and an insect in the order *Hemiptera,* such as the family *Cimicidae.* Examples of insects in the family *Culicidae* include *Anopheles gambiae, Aedes aegypti,* and *Culex quinquefasciatus.* Examples of insects in the family *Drosophilidae* include *Drosophila melanogaster, Drosophila pseudoobscura,* and *Drosophila virilis.* Examples of insects in the family *Bombycidae* include *Bombyx mori, Bombyx mandarina,* and *Trilocha varians.* Examples of insects in the family *Apidae* include *Apis mellifera, Apis florea, Apis dorsata,* and *Bombus terrestris.* Examples of insects in the family *Acrididae* include *Locusta migratoria.* Examples of insects in the family *Cimicidae* include *Cimex lectularius.*

Specifically, examples of wild-type insect odorant receptor proteins include the following: AaOR1, AaOR2, AaOR4, AaOR5, AaOR6, AaOR7, AaOR8, AaOR9, AaOR10a, AaOR15, AaOR22, AaOR24, AaOR25, AaOR26, AaOR27, AaOR28, AaOR30, AaOR34, AaOR36, AaOR38, AaOR41a, AaOR41b, AaOR42, AaOR43, AaOR44, AaOR47, AaOR49, AaOR50, AaOR52, AaOR54, AaOR58, AaOR59, AaOR60, AaOR61, AaOR64, AaOR65, AaOR66, AaOR67a, AaOR69a, AaOR70, AaOR71, AaOR72a, AaOR73, AaOR74, AaOR75, AaOR77, AaOR78, AaOR79, AaOR81, AaOR83b, AaOR84, AaOR85, AaOR86, AaOR87, AaOR91, AaOR95, AaOR97, AaOR96, AaOR99, AaOR100, AaOR102, AaOR103, AaOR104a, AaOR105, AaOR107, AaOR108, AaOR109, AaOR110, AaOR112, AaOR114, AaOR116, AaOR117, AaOR118, AaOR122, AaOR125, AaOR128, AgOR1, AgOR2, AgOR3, AgOR4, AgOR5, AgOR6, AgOR7, AgOR8, AgOR9, AgOR10, AgOR11a, AgOR12a, AgOR12b, AgOR13, AgOR14, AgOR15, AgOR16a, AgOR17, AgOR18, AgOR20, AgOR21, AgOR23, AgOR25, AgOR26, AgOR27, AgOR28, AgOR30, AgOR34, AgOR36, AgOR37, AgOR38, AgOR39a, AgOR40, AgOR42, AgOR44, AgOR45, AgOR46, AgOR47, AgOR49, AgOR50, AgOR54, AgOR56a, AgOR57, AgOR60, AgOR61, AgOR62, AgOR63, AgOR64, AgOR65, AgOR69, AgOR70, AgOR71, AgOR72, AgOR74, AgOR75, AgOR76a, AmOR1, AmOR3, AmOR9, AmOR10, AmOR13, AmOR41, AmOR51, AmOR52, AmOR55, AmOR71, AmOR73, AmOR78, AmOR85, AmOR89, AmOR90, AmOR114, AmOR115, AmOR118, AmOR120, AmOR121, AmOR161, BmOR1, BmOR2, BmOR3, BmOR4, BmOR5, BmOR8, BmOR9, BmOR10, BmOR13, BmOR17, BmOR18, BmOR23, BmOR24, BmOR25, BmOR35, BmOR36, BmOR42, BmOR45, BmOR49, BmOR51, BmOR52, BmOR55, BmOR56, BmOR61, DmOR1a, DmOR9a, DmOR19a, DmOR22a, DmOR22b, DmOR22c, DmOR24a, DmOR30a, DmOR33a, DmOR33b, DmOR33c, DmOR35a, DmOR42b, DmOR43a, DmOR45a, DmOR45b, DmOR47a, DmOR49b, DmOR59b, DmOR65b, DmOR65c, DmOR67b, DmOR67c, DmOR69a, DmOR71a, DmOR74a, DmOR82a, DmOR83a, DmOR83b, DmOR83c, DmOR85a, DmOR85c, DmOR85e, DmOR85f, DmOR88a, DmOR92a, DmOR94a, DmOR94b, and DmOR98b.

In the present specification, OR denotes "odorant receptor." Dm indicates derivation from *Drosophila melanogaster,* Bm indicates derivation from *Bombyx mori,* Ag indicates derivation from *Anopheles gambiae,* and Aa indicates derivation from *Aedes aegypti.* The amino acid sequences of various odorant receptor proteins, including these, and their coding sequences are either known or can be readily identified through sequence identity searches based on known sequences.

The sensor protein may contain one or more amino acid mutations in its wild-type amino acid sequence, provided that the chemical response activity is not significantly decreased. The phrase "not significantly decreased" means, for example, that the chemical response activity of a sensor protein containing amino acid mutations is, for example, at least 50%, preferably at least 60%, more preferably at least 70%, even more preferably at least 80%, and yet more preferably at least 90% of the chemical response activity of the wild-type sensor protein, taken as 100%.

The amino acid mutation is, for example, substitution, insertion, addition, or deletion of an amino acid, preferably substitution, and particularly preferably conservative substitution.

In the present specification, "conservative substitution" refers to the substitution of an amino acid residue with another amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

The sensor protein may contain its wild-type amino acid sequence or an amino acid sequence having, for example, at least 70%, preferably at least 80%, more preferably at least 90%, even more preferably at least 95%, still more preferably at least 98%, and particularly preferably at least 99% identity to the wild-type amino acid sequence.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S., Altschul S. F. Methods for assessing the statistical significance of molecular sequence features by using general scorings schemes, Proc. Natl. Acad. Sci. USA. 87: 2264-2268 (1990); Karlin S., Altschul S. F. Applications and statistics for multiple high-scoring segments in molecular sequences, Proc. Natl. Acad. Sci. USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The identity of base sequences is also determined in the same manner as above.

The sensor protein may have other amino acid sequences, such as protein tags, fluorescent proteins, luminescent proteins, signal sequences, or other proteins or peptides, attached thereto (i.e., the sensor protein in the form of a fusion protein), provided that the chemical response activity is not significantly impaired. Examples of protein tags include biotin, His tag, FLAG tag, Halo tag, MBP tag, HA tag, Myc tag, V5 tag, and PA tag.

In the present specification, chemical response activity refers to the property of a sensor protein to recognize a chemical substance and initiate signal transduction activity (e.g., ion channel activity) either alone or in conjunction with other proteins. In the case of odorant receptors, chemical response activity refers to the property of an odorant receptor to recognize a chemical substance and form an odorant receptor complex, together with an olfactory receptor co-receptor, thereby exhibiting ion channel activity. The chemical response activity of a sensor protein can be measured by using the signal transduction activity of the sensor protein in contact with a chemical substance as an indicator (e.g., by quantifying and evaluating the amount of signal molecules). In the case of odorant receptors, their chemical response activity can be measured using the ion channel activity of an odorant receptor complex formed of an odorant receptor in contact with a chemical substance and an olfactory receptor co-receptor as an indicator. For example, a chemical substance is brought into contact with cells that express a protein that becomes colored or luminescent in response to the influx of ions (e.g. calcium ions) into the cells when (a) an odorant receptor, (b) an olfactory receptor co-receptor, and (c) an odorant receptor complex respond, and the amount of luminescence of the cells is measured. The greater the measured amount of luminescence, the higher the response activity of the odorant receptor to the chemical substance is determined to be. Specifically, the chemical response activity can be measured according to the method described in PTL 1.

The coding sequence for the sensor protein is not particularly limited as long as it is a base sequence encoding the sensor protein.

The expression cassette 1 contains only the coding sequence for the sensor protein as a protein-coding sequence; in other words, the expression cassette 1 does not contain any protein-coding sequences other than the coding sequence for the sensor protein. This enables chemical response activity with enhanced stability regardless of the type of sensor protein. This also enables even higher chemical response activity.

The expression cassette 1 is not particularly limited as long as the expression cassette 1 is a polynucleotide capable of expressing the sensor protein within a cell. The expression cassette 1 contains one promoter and the coding sequence for the sensor protein placed under control of the promoter.

The promoter for the expression cassette 1 is not particularly limited and can be selected as appropriate. The promoter for use can be selected from various types of pol II promoters, for example. Examples of pol II promoters include, but are not particularly limited to, CMV promoter, EF1 promoter, SV40 promoter, MSCV promoter, and promoters derived from insect genes.

Among these promoters, from the perspective of particular suitability as a promoter for the expression cassette 1 (in particular, suitability for expression in insect cells of the family *Arctiidae,* preferably *Spilosoma imparilis* cells, as described below), the PUb promoter is preferred.

The PUb promoter is not particularly limited as long as the PUb promoter is of the polyubiquitin-c gene (NCBI Gene ID: 5579172) derived from *Aedes aegypti* or of its orthologous gene. The orthologous gene is preferably an insect orthologous gene, more preferably an orthologous gene of an insect that belongs to the same family as *Aedes aegypti (Culicidae* family), and even more preferably an orthologous gene of an insect that belongs to the same genus as *Aedes aegypti* (genus of *Aedes).*

The PUb promoter typically includes the transcription start site, its upstream (5') sequence, and optionally, its downstream (3') sequence. When the base at the transcription start site of the above-mentioned gene is designated as +1, with downstream (3' side) bases being considered positive and upstream (5' side) bases being considered 0 or negative, the PUb promoter is any region including the transcription start site within the DNA region of, for instance, -10000 to +500, preferably -5000 to +200, and more preferably -2000 to +150. When multiple transcription start sites are present, the transcription start site with the highest transcription level can be selected. The length of the PUb promoter (number of base pairs: bp) can be, for example, within the range of 500 to 10000 bp, preferably 800 to 7000 bp, more preferably 1000 to 5000 bp, even more preferably 1200 to 3000 bp, and yet more preferably 1200 to 2000 bp.

The PUb promoter may have mutations in its base sequence (e.g., substitutions, deletions, insertions, and additions) as long as the promoter activity does not significantly decrease (e.g., having a promoter activity of 70%, preferably at least 80%, more preferably at least 90%, and even more preferably at least 95%, as compared to the wild type). In this case, the base sequence of the mutated promoter has, for example, at least 70%, preferably at least 80%, more preferably at least 90%, even more preferably at least 95%, still more preferably at least 97%, and particularly preferably at least 99% identity with the corresponding base sequence of the wild-type promoter. It is preferred that the site of mutation be located outside of known expression control elements (e.g., general transcription factor-binding regions and various activator-binding regions). The consensus sequences of expression control elements are already known and can be readily searched in various databases.

The PUb promoter is particularly preferably a base sequence containing a base sequence a represented by SEQ ID NO: 11, or containing a base sequence b having, for example, at least 70%, preferably at least 80%, more preferably at least 90%, even more preferably at least 95%, still more preferably at least 97%, or particularly preferably at least 99% identity with the base sequence a.

The expression cassette 1 preferably contains a terminator sequence downstream of the coding sequence for the sensor protein. There are no particular limitations on the terminator sequence, and terminators derived from various genes can be used. A preferable example of the terminator is SV40 polyA sequence. The SV40 polyA sequence is particularly preferably a base sequence containing base sequence c represented by SEQ ID NO: 10, or containing base sequence d having, for example, at least 70%, preferably at least 80%, more preferably at least 90%, even more preferably at least 95%, still more preferably at least 97%, or particularly preferably at least 99% identity with base sequence c.

From the perspective of enabling chemical response activity with enhanced stability regardless of the type of sensor protein, it is preferred that the distance between the promoter and the coding sequence of the sensor protein be shorter in the expression cassette 1. The base length between the promoter and the coding sequence for the sensor protein is preferably 100 or less, more preferably 50 or less, even more preferably 30 or less, still more preferably 20 or less, and particularly preferably 10 or less. The same also applies when the expression cassette 1 contains a terminator.

It is preferred that a restriction enzyme recognition sequence be arranged on each side of the coding sequence for the sensor protein. This simplifies the replacement of the coding sequence of one sensor protein with that of another sensor protein, enhancing a platform value to exhibit chemical response activity more stably regardless of the type of sensor protein.

The at least two proteins whose coding sequences are included in the expression cassette 2 ("other proteins" below) are not particularly limited as long as they are proteins necessary for chemical detection or drug-resistant gene products in a certain cell. In a preferred embodiment, the expression cassette 2 contains at least two coding sequences selected from the group consisting of a coding sequence for a chromogenic or luminescent protein, a coding sequence for a drug-resistant gene, and a coding sequence for an olfactory receptor co-receptor. In a particularly preferred embodiment, the expression cassette 2 contains a coding sequence for a chromogenic or luminescent protein, a coding sequence for a drug-resistant gene, and a coding sequence for an olfactory receptor co-receptor. From the perspective of chemical response activity, the expression cassette 2 contains, from the 5' end, a coding sequence for a chromogenic or luminescent protein, a coding sequence for a drug-resistant gene, and a coding sequence for an olfactory receptor co-receptor, in this order.

The chromogenic or luminescent protein is not particularly limited as long as it is a protein that becomes colored or luminescent in response to the influx of ions (e.g., calcium ions) into the cell when the sensor protein (in particular, the odorant receptor protein) responds. Examples of such proteins include aequorin, Yellow Cameleon (YC), and GCaMP.

For the drug-resistant gene, a gene resistant to a drug that can be used for drug screening of cells (preferably insect cells) may be selected. Examples of drug-resistant genes include chloramphenicol resistance gene, tetracycline resistance gene, neomycin resistance gene, erythromycin resistance gene, spectinomycin resistance gene, kanamycin resistance gene, hygromycin resistance gene, and puromycin resistance gene.

The olfactory receptor co-receptor (particularly preferably, an insect odorant receptor) is a membrane protein with a seven-transmembrane structure as with the odorant receptor. However, the olfactory receptor co-receptor itself does not recognize odorant substances, but functions by forming a hetero-complex with an odorant receptor. The odorant receptor complex, which is a hetero-complex composed of an odorant receptor and an olfactory receptor co-receptor, has ion channel activity that is activated by odorant substances. When activated, the odorant receptor complex allows the influx of cations such as sodium ions (Na⁺) and calcium ions (Ca²⁺) into cells.

Other proteins may have other amino acid sequences, such as protein tags, fluorescent proteins, luminescent proteins, signal sequences, or other proteins or peptides, attached thereto (i.e., proteins in the form of a fusion protein), provided that the chemical response activity is not significantly impaired. Examples of protein tags include biotin, His tag, FLAG tag, Halo tag, MBP tag, HA tag, Myc tag, V5 tag, and PA tag.

The coding sequences of other proteins are not particularly limited as long as they are base sequences encoding those proteins.

The number of protein-coding sequences included in the expression cassette 2 is, for example, 2 to 5, preferably 2 to 4, more preferably 3 to 4, and particularly preferably 3.

In the expression cassette 2, other sequences are arranged between multiple protein-coding sequences to enable expression of each protein in a non-fused form even with a single promoter. Other sequences include, for example, self-cleaving peptide-coding sequences and IRES (internal ribosome entry site) sequences. However, from the perspective of expression efficiency and chemical response activity, self-cleaving peptide-coding sequences are particularly preferred.

A self-cleaving peptide refers to a peptide sequence with cleavage activity occurring between two amino acid residues within the peptide sequence itself. Examples of self-cleaving peptides include 2A peptides and 2A-like peptides. For example, cleavage in 2A peptides or 2A-like peptides occurs between the glycine residue and the proline residue on these peptides. This occurs due to the "ribosomal skipping mechanism," in which a normal peptide bond does not form between the glycine and proline residues during translation, and does not affect downstream translation. The ribosome skipping mechanism is known in the art and is used for expression of multiple proteins encoded by a single messenger RNA (mRNA) molecule. The self-cleaving peptide used in the present invention can be obtained from a viral 2A peptide or a 2A-like peptide with functionality equivalent to that of a 2A peptide. For example, the self-cleaving peptide can be selected from the group consisting of 2A peptide (F2A) derived from foot-and-mouth disease virus (FMDV), 2A peptide (E2A) derived from equine rhinitis A virus (ERAV), 2A peptide (P2A) derived from Porcine teschovirus (PTV-1), and 2A peptide (T2A) derived from Thosea asigna virus (TaV). For example, T2A having the amino acid sequence of SEQ ID NO: 6 and P2A having the amino acid sequence of SEQ ID NO: 4 are usable. 2A peptides are reviewed in Expert Opinion on Biological Therapy, volume 5, pages 627-638, 2005. In an embodiment of the present disclosure, the self-cleaving peptide is a peptide composed of the amino acid sequence of SEQ ID NO: 4 or 6, or a peptide that is composed of an amino acid sequence having one or more (for example, 2 to 3, preferably 2) amino acids substituted, deleted, added, or inserted (preferably conservatively substituted) in the amino acid sequence of SEQ ID NO: 4 or 6, and that has self-cleaving activity not significantly impaired (e.g., exhibiting activity of 50%, 70%, 90%, 95%, or 99% or higher relative to the wild-type). Additionally, from the perspective of expression efficiency and chemical response activity, the self-cleaving peptide is preferably the aforementioned, multiple (for example, 2 to 4, preferably 2 to 3, and particularly preferably 2) self-cleaving peptides linked in tandem.

In the expression cassette 2 containing a self-cleaving peptide-coding sequence, it is preferred that the distance between the self-cleaving peptide-coding sequence and the protein-coding sequence on each side of the self-cleaving peptide-coding sequence be shorter. The base length between the self-cleaving peptide-coding sequence and the protein-coding sequence on each side is preferably 100 or less, more preferably 50 or less, still more preferably 30 or less, even more preferably 20 or less, yet more preferably 10 or less, and particularly preferably 0.

The expression cassette 2 is not particularly limited as long as it is a polynucleotide capable of expressing multiple other proteins in a non-fused form in a cell. The expression cassette 2 contains one promoter and coding sequences for other proteins placed under control of the promoter.

The promoter for the expression cassette 2 is not particularly limited and can be selected as appropriate. The promoter for use can be selected, for example, from various types of pol II promoters. Examples of pol II promoters include, but are not limited to, CMV promoter, EF1 promoter, SV40 promoter, MSCV promoter, and promoters derived from insect genes.

Among these promoters, from the perspective of particular suitability as a promoter for the expression cassette 2 (in particular, suitability for expression in insect cells of the family *Arctiidae,* preferably *Spilosoma imparilis* cells, as described below), the 121 promoter is preferred.

The 121 promoter is not particularly limited as long as the 121 promoter is of the g7775 gene derived from *Polypedilum vanderplanki* (Int. J. Mol. Sci. 2021 May 28; 22(11): 5798. doi: 10.3390/ijms22115798. PMID: 34071490; PMCID: PMC8197945) or of its orthologous gene. The orthologous gene is preferably an insect orthologous gene, more preferably an orthologous gene of an insect that belongs to the same family as *Polypedilum vanderplanki* (Chironomidae family), and even more preferably an orthologous gene of an insect that belongs to the same genus as *Polypedilum vanderplanki* (genus of *Polypedilum*)*.*

The 121 promoter typically includes the transcription start site, its upstream (5') sequence, and optionally, its downstream (3') sequence. When the base at the transcription start site of the above-mentioned gene is designated as +1, with downstream (3' side) bases being considered positive and upstream (5' side) bases being considered 0 or negative, the 121 promoter is any region including the transcription start site within the DNA region of, for instance, -10000 to +500, preferably -5000 to +200, and more preferably -2000 to +150. When multiple transcription start sites are present, the transcription start site with the highest transcription level can be selected. The length of the 121 promoter (number of base pairs: bp) can be, for example, within the range of 500 to 10000 bp, preferably 800 to 7000 bp, more preferably 1000 to 5000 bp, even more preferably 1200 to 3000 bp, and yet more preferably 1200 to 2000 bp.

The 121 promoter may have mutations in its base sequence (e.g., substitutions, deletions, insertions, and additions) as long as the promoter activity does not significantly decrease (e.g., having a promoter activity of 70%, preferably at least 80%, more preferably at least 90%, and even more preferably at least 95%, as compared to the wild type). In this case, the base sequence of the mutated promoter has, for example, at least 70%, preferably at least 80%, more preferably at least 90%, even more preferably at least 95%, still more preferably at least 97%, and particularly preferably at least 99% identity with the corresponding base sequence of the wild-type promoter. It is preferred that the site of mutation be located outside of known expression control elements (e.g., general transcription factor-binding regions and various activator-binding regions). The consensus sequences of expression control elements are already known and can be readily searched in various databases.

The 121 promoter is particularly preferably a base sequence containing a base sequence e represented by SEQ ID NO: 1, or containing a base sequence f having, for example, at least 70%, preferably at least 80%, more preferably at least 90%, even more preferably at least 95%, still more preferably at least 97%, or particularly preferably at least 99% identity with the base sequence e.

The expression cassette 2 preferably contains a terminator sequence downstream of the coding sequence for the sensor protein. For the terminator sequence, the same description as that for the expression cassette 1 applies.

In the expression cassette 2, it is preferred that the distance between the promoter and the protein-coding sequence closest to the promoter be shorter. The base length between the promoter and this protein-coding sequence is preferably 100 or less, more preferably 50 or less, even more preferably 30 or less, still more preferably 20 or less, yet more preferably 10 or less, and particularly preferably 0.

In expression cassette 2 containing a terminator, it is preferred that the distance between the terminator and the protein-coding sequence closest to the terminator be shorter. The base length between the terminator and this protein-coding sequence is preferably 100 or less, more preferably 50 or less, even more preferably 30 or less, still more preferably 20 or less, yet more preferably 10 or less, and particularly preferably 0.

In the present specification, the polynucleotide include not only typical polynucleotides such as DNA and RNA inherent in organisms, but also polynucleotides with known chemical modifications, artificial polynucleotides, and like polynucleotides, as listed below. To prevent degradation due to hydrolases such as nucleases, the phosphate residue of each nucleotide can be substituted with a chemically modified phosphate residue, such as phosphorothioate (PS), methylphosphonate, or phosphorodithionate. The hydroxyl group at position 2 of the ribose of each ribonucleotide may also be substituted with -OR (R indicates, for example, CH₃(2'-O-Me), CH₂CH₂OCH₃(2'-O-MOE), CH₂CH₂NHC(NH)NH₂, CH₂CONHCH₃, or CH₂CH₂CN). Additionally, the nucleobase moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl moiety, for example, with biotin, an amino group, a lower alkyl amine group, or an acetyl group. The polynucleotide for use can also be, for example, BNA (LNA), which is prepared by crosslinking the 2' oxygen and the 4' carbon in the ribose moiety of a nucleotide to fix the ribose moiety in N-conformation.

In addition to the expression cassette 1 and the expression cassette 2, the polynucleotide of the present disclosure may optionally contain other elements (e.g., restriction enzyme recognition sequences, multiple cloning sites (MCS), replication origins, enhancer sequences, and insulator sequences).

The polynucleotide of the present disclosure is a single molecule. In other words, a combination of a polynucleotide molecule containing the expression cassette 1 and another polynucleotide molecule containing the expression cassette 2 is composed of two molecules and does not correspond to the polynucleotide of the present disclosure.

The polynucleotide of the present disclosure can be single-stranded or double-stranded, and can be linear or annular.

From the viewpoint of direct use in introduction into cells and direct use in the production of sensor cells, the polynucleotide of the present disclosure is preferably a vector (expression vector). Additionally, for the production of cells with stable expression, it is preferred that the vector be a transposon vector. The type of vector is not particularly limited, and known vectors can be used.

In an embodiment, the present disclosure relates to a cell comprising the polynucleotide of the present disclosure ("the cell of the present disclosure" in the present specification).

The cell is not particularly limited. From the perspective of chemical detection suitability, the cell is preferably animal cells such as insect cells or mammalian cells, with insect cells being particularly preferred. Among insect cells, those derived from insects of the family *Arctiidae* are particularly preferred.

The cells derived from insects of the family *Arctiidae* are not particularly limited as long as they are primary cultured cells or established cell lines of biological constituent cells derived from insects of the family *Arctiidae.*

Examples of the family *Arctiidae* include subfamilies such as *Arctiinae, Lithosiinae,* and *Syntominae,* among which the subfamily *Arctiinae* is preferred. The subfamily *Arctiinae* is, for example, preferably the genus *Spilosoma, Spilarctia,* or *Rhagonis,* with the genus *Spilosoma* being particularly preferable. While there is no particular limitation to the genus *Spilosoma, Spilosoma imparilis* is particularly preferable.

The cells derived from insects of the family *Arctiidae* can be obtained from known biological banks or collected and cultured from living insects of the family *Arctiidae* according to or with reference to known methods; if necessary, they can be established as cell lines.

Examples of cells derived from *Spilosoma imparilis* include FFPRI-SpIm-2AM-SF cells (MAFF No.: 275052) and FFPRI-SpIm-2AM-IPL411 cells (MAFF No.: 275053) from the Genebank Project, National Agriculture and Food Research Organization.

In the cell of the present disclosure, the polynucleotide of the present disclosure is preferably integrated into the genomic DNA (particularly preferably chromosomal genomic DNA). This enables stable expression of the sensor protein, making it suitable for chemical detection. In this case, the polynucleotide of the present disclosure can be a single continuous region within the genomic DNA. In another embodiment, the polynucleotide of the present disclosure in the cell of the present disclosure may be present in a state where it is not integrated into the genomic DNA.

From the perspective of suitability for use in chemical sensors, the cell of the present disclosure is preferably incorporated in a cell chip. Thus, in an embodiment, the present disclosure relates to a cell chip including one or more compartments each containing the cell of the present disclosure ("the cell chip of the present disclosure" in the present specification).

The compartments of the cell chip of the present disclosure preferably contain cells and hydrogel. This protects the cells from desiccation.

The configuration of the compartments is not particularly limited as long as it is capable of retaining the cells. From the perspectives of cell retention, production efficiency, or chemical detection, the compartments are preferably in the form of wells.

The material of the compartments is not particularly limited as long as the material can retain cells. The material can be, for example, resin or metal.

From the perspective of detection sensitivity, a compartment typically contains multiple cells. The number of cells per unit area (cm²) in a compartment is, for example, 1×10³ to 1×10⁹ cells/cm².

From the perspective of detection sensitivity or production efficiency, the bottom surface area of a single compartment is preferably 0.5 to 100 mm², more preferably 1 to 30 mm², and even more preferably 1.5 to 10 mm².

From the perspective of detection sensitivity or production efficiency, the number of compartments included in the cell chip is preferably 10 to 2000, more preferably 30 to 1000, and even more preferably 50 to 500.

The cell of the present disclosure is suitable for detecting chemical substances using various sensor proteins. Thus, it is preferred that the cell chip of the present disclosure includes two or more (more preferably three or more, even more preferably four or more, still more preferably five or more, ten or more, 15 or more, or 20 or more) types of cells that differ from each other in the type of sensor protein.

The cell of the present disclosure and the cell chip of the present disclosure can be used in the detection of chemical substances (in particular, odorous substances). The chemical substances can be, for example, those in a sample such as body fluids (e.g., urine, blood, and saliva), air (e.g., indoor air and air inside packaging), and water (e.g., river water, seawater, tap water, clean water, and sewage). In this case, for example, the cell of the present disclosure is brought into contact with the sample, or the sample is added to compartments of the cell chip of the present disclosure; this allows the chemical substance in the sample to reach the cells and come into contact with the sensor protein in the cells. For example, a chemical substance can be detected by detecting ions flowing into the cells (e.g., by detecting a protein that becomes colored or luminescent in response to ions).

### Examples

The present invention will be described in detail below with reference to Examples. However, the present invention is not limited to these Examples.

### Test Example 1: Chemical Response Activity Evaluation Test 1

### (Comparative Example)

A vector containing an odorant receptor protein as a sensor protein was designed; the vector included the coding sequence for the odorant receptor protein, the coding sequence for an olfactory receptor co-receptor, the coding sequence for a calcium sensor photoprotein, and the coding sequence for a puromycin resistance gene. To keep the vector size small by limiting the number of promoters and terminators to improve the efficiency of vector delivery into cells, multiple protein-coding sequences were incorporated into a single expression cassette, with the coding sequence for a 2A peptide (self-cleaving peptide) placed between the coding sequences. Specifically, the vector was designed according to embodiment X and embodiment Y in Fig. 1.

The element indicated in each box in Fig. 1 is as described below. In Fig. 1, the bars between boxes represent spacer sequences.

### 5' ITR

### 5' Inverted terminal repeat

### 121

121 promoter (promoter of g7775 gene derived from *Polypedilum vanderplanki* (Int. J. Mol. Sci. 2021 May 28; 22(11): 5798. doi: 10.3390/ijms22115798. PMID: 34071490; PMCID: PMC8197945), base sequence: SEQ ID NO: 1)

### GcaMP7s

Calcium sensor photoprotein (amino acid sequence: SEQ ID NO: 2) coding sequence (base sequence: SEQ ID NO: 3)

### 2A

A sequence in which the coding sequence (base sequence: SEQ ID NO: 5) of the self-cleaving peptide P2A (amino acid sequence: SEQ ID NO: 4) and the coding sequence (base sequence: SEQ ID NO: 7) of another self-cleaving peptide T2A (amino acid sequence: SEQ ID NO: 6) are linked in tandem

### PuroR

The coding sequence (base sequence: SEQ ID NO: 9) of puromycin resistance gene (amino acid sequence: SEQ ID NO: 8)

### SV40

SV40 polyA sequence (base sequence: SEQ ID NO: 10)

### PUb

PUb promoter (promoter of the polyubiquitin-c gene (NCBI Gene ID: 5579172) derived from *Aedes aegypti,* base sequence: SEQ ID NO: 11)

### OR

The coding sequence of any of the following odorant receptor proteins:
AaOR47 (amino acid sequence: SEQ ID NO: 12, base sequence of the coding sequence: SEQ ID NO: 13), and ORA, ORB

### Orco

The coding sequence (base sequence: SEQ ID NO: 15) for an olfactory receptor co-receptor (amino acid sequence: SEQ ID NO: 14)

### 3' ITR

### 3' Inverted terminal repeat

The vector of embodiment X and the vector of embodiment Y were individually introduced into cells derived from *Spilosoma imparilis* (SpIm cells). After 72 hours, a chemical response activity evaluation test was conducted. The method of the test is as described below. Vector-introduced cells were seeded in a 96-well plate (CORNING 3909) at a density of 1 × 10⁵ cells/200 µL/well and left to stand in a 27°C incubator (without CO₂ supply). The culture medium used was Sf-900III SFM medium. Cell counting was performed using a Countess II FL Automated Cell Counter (Thermo Fisher) after the cell suspension was stained with trypan blue. After 24 hours from seeding, the culture fluid was removed from the 96-well plate and replaced with 80 mL of 0.1% BSA HBSS (-) (without phenol red). After five minutes from this fluid exchange operation, the fluorescence intensity (GCaMP) was measured using a FlexStation 3 microplate reader. Specifically, chemical substances (substances to which odorant receptors respond) adjusted to various concentrations (0, 0.01, 0.1, 1, 10, and 100 µM) were added to the wells, and changes in fluorescence intensity before and after addition of chemical substances were quantified using a microplate reader (FlexStation3, Molecular Devices).

Figs. 2 to 4 shows the results. As shown in Figs. 2 to 4, incorporating a sensor protein-coding sequence and coding sequences for other proteins into a single expression cassette resulted in the position of the sensor protein-coding sequence affecting the chemical response activity, and this impact varied depending on the type of sensor protein. Given that depending on the position, some type of sensor protein did not exhibit chemical response activity (embodiment X in Fig. 4), the embodiment of incorporating a sensor protein-coding sequence and coding sequences for other proteins within a single expression cassette was found to be unsuitable as a platform for using multiple types of sensor proteins.

### Test Example 2: Chemical Response Activity Evaluation Test 2

### (Example)

Based on the results of Test Example 1, a decision was made to not link the sensor protein-coding sequence with the coding sequences of other proteins. As in Test Example 1, in order to keep the vector size small by limiting the number of promoters and terminators to thereby improve the efficiency of vector delivery into cells, multiple coding sequences were determined to be placed in another expression cassette. Specifically, a vector was designed according to embodiment Z in Fig. 5.

The elements indicated in the boxes in Fig. 5 are the same as those in Test Example 1.

SpIm cells were transfected with the vector of embodiment X, embodiment Y, or embodiment Z (all transposon vectors), and selection was performed with puromycin, thereby preparing stable-expression cells having exogenous DNA integrated into the chromosomal genomic DNA. A chemical response activity evaluation test was conducted using the cells in the same manner as in Test Example 1.

Fig. 6 shows some of the results. Not linking the sensor protein-coding sequence with the coding sequences for other proteins eliminates the effect on chemical response activity (which possibly leads to inactivation in some cases) that occurs due to the position of the sensor protein-coding sequence incorporated together with the coding sequences for other proteins into a single expression cassette.

## Claims

1. A polynucleotide comprising
an expression cassette 1 containing only a coding sequence for a sensor protein as a protein-coding sequence, and
an expression cassette 2 containing at least two coding sequences for proteins.

2. The polynucleotide according to claim 1, wherein the sensor protein is an odorant receptor protein.

3. The polynucleotide according to claim 1, wherein the sensor protein is an insect odorant receptor protein.

4. The polynucleotide according to claim 1, wherein the expression cassette 2 contains at least two coding sequences selected from the group consisting of a coding sequence for a chromogenic or luminescent protein, a coding sequence for a drug-resistant gene, and a coding sequence for an olfactory receptor co-receptor.

5. The polynucleotide according to claim 1, wherein the expression cassette 2 contains a coding sequence for a chromogenic or luminescent protein, a coding sequence for a drug-resistant gene, and a coding sequence for an olfactory receptor co-receptor.

6. The polynucleotide according to claim 1, wherein the expression cassette 2 has a self-cleaving peptide-coding sequence inserted between the coding sequences.

7. The polynucleotide according to claim 1, which is a vector.

8. A cell comprising the polynucleotide of any one of claims 1 to 7.

9. A cell chip comprising one or more compartments each containing the cell of claim 8.

10. The cell chip according to claim 9, wherein the cell includes two or more types of cells containing mutually different types of the sensor protein.

11. The cell chip according to claim 9, for use in the detection of a chemical substance.
